# EUROPEAN PATENT APPLICATION

(11) **EP 4 289 947 A1**
(43) Date of publication of application: **13.12.2023**
(21) Application number: 22749122.2
(22) Date of filing: 28.01.2022
(51) Int. Cl.: C12N 9/10, C12N 15/01, C12N 15/09

(54) **HIGH-STEREOSELECTIVITY R TRANSKETOLASE MUTANT, AND ENCODING GENE AND USE THEREOF**

(30) Priority: 05.02.2021 CN 202110162619; 24.05.2021 CN 202110567238
(71) Applicant: Zhejiang Apeloa Kangyu Pharmaceutical Co. Ltd., Zhejiang 322118 (CN); Apeloa Pharmaceutical Co., Ltd., Zhejiang 322118 (CN)
(72) Inventor: LIN, Shuangjun, Shanghai 200240 (CN); LIU, Qi, Shanghai 200240 (CN); DENG, Zixin, Shanghai 200240 (CN); HUANG, Tingting, Shanghai 200240 (CN)
(74) Representative: Perani & Partners S.p.A.
(86) International application number: PCT/CN2022/074751
(87) International publication number: WO 2022/166843

(57) **Abstract**

Provided are a transketolase mutant, and an encoding gene and the use thereof, relating to the technical field of enzyme engineering. The transketolase mutant provided in the present invention has a sequence as set forth in SEQ ID NO:16 with an amino acid mutation. Provided are a high-stereoselectivity R transketolase mutant with an amino acid sequence of SEQ ID NO:1, a recombinant expression vector containing the encoding gene, a host cell containing the recombinant expression vector, a method for preparing the high-stereoselectivity R transketolase mutant, the use of the R transketolase mutant in catalyzing aromatic aldehyde to form high-stereoselectivity R-aromatic dihydroxyketone, and the use of the encoding gene in catalyzing aromatic aldehyde to form high-stereoselectivity R-aromatic dihydroxyketone. The stereoselectivity of p-methylsulfonephenyl dihydroxyketone produced under the catalysis of the mutant EcTK1_YYH is 95.2% ee (R), and a high activity and high steroselectivity are achieved.

## Description

The present invention claims the priority of the Chinese patent application submitted on Feb. 5, 2021 (Application No.: 202110162619.2. Invention name: High-Stereoselectivity R Transketolase Mutant, and Encoding Gene and Use Thereof, and on May. 24, 2021 (Application No.: 202110567238.2. Invention name: Method for Synthesizing (1R, 2R) - AMPP By Using Enzyme Cascade Reaction), the contents of which are incorporated into the present invention by reference.

### Field of the Invention

The present invention relates to the enzyme engineering technology and a transketolase mutant, i.e., an E. coli transketolase mutant and its encoding gene and application, particularly to a high-stereoselectivity R transketolase mutant, and encoding gene and use thereof.

### Background of the Invention

Transketolase is a ubiquitous thiamine pyrophosphate-dependent enzyme. It can promote the non-oxidative pentose phosphate pathway and the tricarboxylic acid cycle. Transketolase can assist in catalyzing a reversible transketo reaction, which transfers the two-carbon-atom unit of the keto alcohol donor to an aldehyde receptor to generate a chiral dihydroxyketone compound. In the transketo reaction, β-hydroxypyruvate is usually used as a ketone alcohol donor because β- hydroxypyruvate can generate volatile carbon dioxide after transketo, making the reaction irreversible. Recently, methods for detecting dihydroxyketone compounds, such as colorimetric and HPLC have been published. Additionally, chiral GC and HPLC methods for determining the enantioselectivity of dihydroxyketone compounds have been established. Establishing these detection methods is beneficial for modifying the catalytic activity and stereoselectivity of transketolase mutants.

The enantiomer excess *ee* reflects the stereoselectivity, which indicates a chiral compound's optical purity. The higher the *ee* value, the higher the optical purity. In 2008, Helen C. Hailes' research team reported that wild E. coli transketolase could catalyze L-erythrulose formation with a high stereoselectivity of 95%. The enantiomer excess *ee*(*S*) indicates the optical purity of a chiral compound. The higher the *ee* value, the higher the optical purity. However, it can catalyze the formation of moderately stereoselective hydroxyketone products from valeraldehyde. Some studies have enhanced and reversed the stereoselectivity of valeraldehyde catalyzed by transketolase through site-directed mutagenesis of D469 and H26. The S selectivity of D469E was enhanced (ee (*S*) = 90%), while the H26Y mutant catalyzed the formation of (*R*)-hydroxyketone (ee = 88%) [Enhancing and Reversing the Stereoselectivity of Escherichia coli Transketolase via Single-Point Mutations, Adv. Synth. Catal. 2008, 350, 2631 - 2638]. In 2019, Paul A. Dalby's research team reported that the mutant screened and obtained through saturation mutation and combination mutation of transketolase from E. coli could help to catalyze pyruvic acid donor. When valeraldehyde and caproaldehyde serve as receptors, a high stereoselectivity (*S*) - hydroxyketone (ee>98%) can be produced [Engineering transketolase to accept both unnatural donor and acceptor substrates and produce α-hydroxyketones, The FEBS Journal 287 (2020) 1758-1776]. In conclusion, transketolase has high catalytic activity for aliphatic aldehydes (e.g., propionaldehyde, valeraldehyde, and caproaldehyde). Moreover, the transketolase mutant obtained through enzyme evolution has a high S selectivity (*ee* = 90% -98%) and *R* selectivity (*ee* = 88%).

In addition to catalyzing aliphatic aldehydes, transketolase can also catalyze aromatic aldehydes after modification. In 2010, Helen C. Hailes' research team reported that Site-directed mutagenesis of D469 and F434 was carried out for E. coli transketolase from E. coli, achieving the catalysis of benzaldehyde and m-hydroxy benzaldehyde. However, the catalytic efficiency was low, and the transformation ratio was ≤ 10%. The hydroxylketone products from their mutant catalysis were mainly in the R-configuration (*ee* ≤ 82%) [α, α'-Dihydroxyketone formation using aromatic and heteroaromatic aldehydes with evolved transketolase enzymes, Chem. Commun., 2010, 46, 7608-7610]. Paul A. Dalby's research team stated in 2015 that R520 and S385 iterative saturation mutations were performed using E. coli TK/D469T as the basis. In addition, the screened mutant *Ec*TK/D469T/R520Q/S385Y had a high catalytic activity to benzaldehyde derivatives, including m-carboxy benzaldehyde, m-hydroxy benzaldehyde, and p-hydroxy benzaldehyde while broadening the substrate spectrum of the E. coli transketolase. However, its stereoselectivity is unknown. [Second-generation engineering of transketolase for polar aromatic aldehyde substrates, Enzyme and Microbial TecH26ology 71 (2015) 45-52]. In 2017, Wolf-Dieter Fessner's research team revealed that the catalytic efficiency of transketolase for phenylacetaldehyde, phenyl propionaldehyde, phenoxy acetaldehyde, and benzyloxy acetaldehyde was increased through the guided evolution of transketolase from Bacillus stearothermophilus. The yield was 60-72%, and its hydroxyketone product had an absolute *S*-stereoselectivity (*ee* > 99%). In addition, the transketolase mutant L382N/D470S obtained through directed evolution has a high catalytic activity for benzaldehyde substrate. However, its stereoselectivity is unknown [Second-generation engineering of transketolase for polar aromatic aldehyde substrates, Green Chem., 2017, 19, 481-489]. In conclusion, as reported in the literature, the catalytic activity of transketolase for aromatic aldehydes has been improved through the modification of transketolase, which catalyzes the production of hydroxyketone products with S-stereoconfiguration from aromatic aldehyde substrates like phenyl acetaldehyde, phenyl propionaldehyde, and phenoxy acetaldehyde. In contrast, the product configuration of catalyzed benzaldehyde is unknown. Therefore, no transketolase that can efficiently catalyze aromatic aldehydes, especially benzaldehyde derivatives, to produce *R*-configuration hydroxyketone products in literature reports.

The high-stereoselectivity *R*-aromatic dihydroxyketone is a crucial synthetic block for ketose, chiral amino diol, and other high-value compounds. For example, *R*-p-methyl sulfonyl phenyl dihydroxyketone is a chiral intermediate of thiamphenicol and florfenicol. *R*-p-nitro phenyl dihydroxyketone is a chiral intermediate of chloramphenicol. *R*-phenyl hydroxy ketone compound is a chiral intermediate of phenylpropanolamine and norpseudoephedrine. Therefore, the *R* transketolase mutant mentioned in the present invention can be used for the biosynthesis of chiral drug intermediates and has a specific application value.

Therefore, technicians in this field are committed to developing a high activity and stereoselectivity *R* transketolase, which can be applied to synthesize *R*-aromatic dihydroxyketone, improve the substrate transformation ratio and the product's optical purity *R*-aromatic dihydroxyketone and meet the requirements of the industrial production accordingly.

### Description of the Invention

Considering the above limitations of existing technology, the technical problem to be solved by the present invention is how to obtain a high activity and stereoselectivity *R* transketolase that can be applied to synthesize *R*-aromatic dihydroxyketone, improve the substrate transformation ratio and the product's optical purity *R*-aromatic dihydroxyketone and meet the requirements of the industrial production accordingly.

The present invention provides the following technical solutions:
[1]. A transketolase mutant with amino acid mutation sequence as represented by SEQ ID NO: 16, and the said amino acid mutation is H26Y or one of the following combinatorial mutation sites: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H261G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26Y+F434Y+L466H+H461R.
[2]. The transketolase mutant described according to [1], where the transketolase mutant is the transketolase mutant derived from E. coli.
[3].The transketolase mutant described according to [1] or [2], where the transketolase mutant is an *R* transketolase mutant.
[4]. The transketolase mutant described according to any item within [1]-[3] is a high-stereoselectivity *R* transketolase mutant with an amino acid sequence as represented by SEQ ID NO: 1.
[5]. The transketolase mutant described according to [4], where the nucleotide sequence of the encoding gene is that as represented by SEQ ID NO: 2.
[6]. The recombinant expression vector containing the transketolase's encoding gene mutant described in [5].
[7]. Host cells with the recombinant expression vector containing the transketolase's encoding gene mutant described in [6].
[8]. A method for preparing the ketotransferase mutant described according to any item within [1]-[5], where the said method includes the following steps:
   Step 1: With the gene derived from prokaryotic E. coli transketolase as a template, obtain the high-stereoselectivity *R* transketolase mutant DNA molecules through PCR site-directed mutation and saturation mutation.
   Step 2: Construct a recombinant expression vector containing the high-stereoselectivity *R* transketolase mutant DNA molecules obtained according to the Step 1.
   Step 3: Through IPTG induction, produce and obtain a large amount of high-stereoselectivity *R* transketolase mutant with biological activity from the host cells containing the recombinant expression vector obtained according to the Step 2.
   Step 4: Obtain high activity and stereoselectivity *R* transketolase mutant protein through affinity chromatography by separating and purifying the high-stereoselectivity *R* transketolase mutant protein with biological activity obtained according to the Step 3.
[9]. According to [8], the method described where the said transketolase mutant is generated by the amino acid sequence of the said prokaryotic E. coli transketolase through the said site-directed mutation and iterative saturation mutation. The serine at Site 385 of the amino acid sequence of the said prokaryotic E. coli transketolase is mutated into tyrosine. The aspartic acid at Site 469 is mutated into threonine. The arginine at Site 520 is mutated into glutamine. The histidine at Site 26 is mutated into tyrosine. The phenylalanine at Site 434 is mutated into tyrosine. The leucine at Site 466 is mutated into histidine.
[10]. The application of the transketolase mutant described according to any item within [1]-[4] catalyzes the formation of high-stereoselectivity *R*-aromatic dihydroxyketone from aromatic aldehyde.
[11]. The application of the transketolase's encoding gene mutant described according to [5] catalyzes the formation of high-stereoselectivity *R*-aromatic dihydroxyketone from aromatic aldehydes.
[12]. The application described according to [10], wherein, the p-methyl sulfonyl benzaldehyde as the substrate and transketolase mutant as the catalytic enzyme, the said p-methyl sulfonyl benzaldehyde could be synthesized into *R*-p-methyl sulfonyl phenyl dihydroxyketone.
[13] According to [11], the application described where a recombinant expression vector containing the encoding gene of the high-stereoselectivity *R* transketolase mutant can be constructed. The host cells containing the said recombinant expression vector can produce a large amount of high-stereoselectivity *R* transketolase mutant with biological activity through IPTG induction. The high-stereoselectivity *R* transketolase mutant protein with biological activity can be separated and purified through affinity chromatography to obtain the said high activity and stereoselectivity *R* transketolase mutant protein. With the p-methyl sulfonyl benzaldehyde as the substrate and the said high activity and stereoselectivity *R* transketolase mutant protein as the catalytic enzyme, the said p-methyl sulfonyl benzaldehyde could be synthesized into the said *R*-p-methyl sulfonyl phenyl dihydroxyketone.

Further, to achieve the above purpose, the present invention with an E. coli transketolase mutant TK/D469T/R520Q/S385Y reported in the literature as the female parent (its sequence is represented by SEQ ID NO: 16) and through three rounds of saturation mutation, provides highly efficient catalytic benzaldehyde derivatives, including p-methyl sulfonyl benzaldehyde, p-fluoro benzaldehyde, p-chloro benzaldehyde, p-bromo benzaldehyde, and p-nitro benzaldehyde. P-methyl sulfonyl benzaldehyde and benzaldehyde can produce transketolase mutants of R-hydroxy ketones, e.g., EcTK1_YYH.

In some Embodiments of the present invention, an E. coli transketolase mutant is provided. It has an amino acid mutation sequence represented by SEQ ID NO: 16, and the said amino acid mutation Site is H26Y or one of the following combinatorial mutation Sites: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H261G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26Y+F434Y+L466H+H461R.

Some Embodiments of the present invention provide a high-stereoselectivity *R* transketolase mutant. Its amino acid sequence is represented by SEQ ID NO: 1 (based on the female parent of E. coli transketolase mutant TK/D469T/R520Q/S385Y Amino acid mutation Site: H26Y+F434Y+L466H). The nucleotide sequence of its encoding gene is represented by SEQ ID NO: 2.

The sequences represented by SEQ ID NO: 1 are as follows:

MSSRKELANA IRALSMDAVQ KAKSGYPGAP MGMADIAEVL WRDFLKHNPQ NPSWADRDRF 60
VLSNGHGSML IYSLLHLTGY DLPMEELKNF RQLHSKTPGH PEVGYTAGVE TTTGPLGQGI 120
ANAVGMAIAE KTLAAQFNRP GHDIVDHYTY AFMGDGCMME GISHEVCSLA GTLKLGKLIA 180
FYDDNGISID GHVEGWFTDD TAMRFEAYGW HVIRDIDGHD AASIKRAVEE ARAVTDKPSL 240
LMCKTIIGFG SPNKAGTHDS HGAPLGDAEI ALTREQLGWK YAPFEIPSEI YAQWDAKEAG 300
QAKESAWNEK FAAYAKAYPQ EAAEFTRRMK GEMPSDFDAK AKEFIAKLQA NPAKIASRKA 360
SQNAIEAFGP LLPEFLGGSA DLAPYNLTLW SGSKAINEDA AGNYIHYGVR EFGMTAIANG 420
ISLHGGFLPY TSTYLMFVEY ARNAVRMAAL MKQRQVMVYT HDSIGHGETG PTHQPVEQVA 480
SLRVTPNMST WRPCDQVESA VAWKYGVERQ DGPTALILSQ QNLAQQERTE EQLANIARGG 540
YVLKDCAGQP ELIFIATGSE VELAVAAYEK LTAEGVKARV VSMPSTDAFD KQDAAYRESV 600
LPKAVTARVA VEAGIADYWY KYVGLNGAIV GMTTFGESAP AELLFEEFGF TVDNVVAKAK 660
ELL 663

The sequences represented by SEQ ID NO: 2 are as follows:

atgtcctcac gtaaagagct tgccaatgct attcgtgcgc tgagcatgga cgcagtacag 60
aaagccaaat ccggttatcc gggtgcccct atgggtatgg ctgacattgc cgaagtcctg 120
tggcgtgatt tcctgaaaca caacccgcag aatccgtcct gggctgaccg tgaccgcttc 180
gtgctgtcca acggccacgg ctccatgctg atctacagcc tgctgcacct caccggttac 240
gatctgccga tggaagaact gaaaaacttc cgtcagctgc actctaaaac tccgggtcac 300
ccggaagtgg gttacaccgc tggtgtggaa accaccaccg gtccgctggg tcagggtatt 360
gccaacgcag tcggtatggc gattgcagaa aaaacgctgg cggcgcagtt taaccgtccg 420
ggccacgaca ttgtcgacca ctacacctac gccttcatgg gcgacggctg catgatggaa 480
ggcatctccc acgaagtttg ctctctggcg ggtacgctga agttgggtaa actgattgca 540
ttctacgatg acaacggtat ttctatcgat ggtcacgttg aaggctggtt caccgacgac 600
accgcaatgc gtttcgaagc ttacggctgg cacgttattc gcgacatcga cggtcatgac 660
gcggcatcta tcaaacgcgc agtagaagaa gcgcgcgcag tgactgacaa accttccctg 720
ctgatgtgca aaaccatcat cggtttcggt tccccgaaca aagccggtac ccacgactcc 780
cacggtgcgc cgctgggcga cgctgaaatt gccctgaccc gcgaacaact gggctggaaa 840
tatgcgccgt tcgaaatccc gtctgaaatc tatgctcagt gggatgcgaa agaagcaggc 900
caggcgaaag aatccgcatg gaacgagaaa ttcgctgctt acgcgaaagc ttatccgcag 960
gaagccgctg aatttacccg ccgtatgaaa ggcgaaatgc cgtctgactt cgacgctaaa 1020
gcgaaagagt tcatcgctaa actgcaggct aatccggcga aaatcgccag ccgtaaagcg 1080
tctcagaatg ctatcgaagc gttcggtccg ctgttgccgg aattcctcgg cggttctgct 1140
gacctggcgc cgtataacct gaccctgtgg tctggttcta aagcaatcaa cgaagatgct 1200
gcgggtaact acatccacta cggtgttcgc gagttcggta tgaccgcgat tgctaacggt 1260
atctccctgc acggtggctt cctgccgtac acctccacct atctgatgtt cgtggaatac 1320
gcacgtaacg ccgtacgtat ggctgcgctg atgaaacagc gtcaggtgat ggtttacacc 1380
cacgactcca tcggtcatgg cgaaaccggc ccgactcacc agccggttga gcaggtcgct 1440
tctctgcgcg taaccccgaa catgtctaca tggcgtccgt gtgaccaggt tgaatccgcg 1500
gtcgcgtgga aatacggtgt tgagcgtcag gacggcccga ccgcactgat cctctcccaa 1560
cagaacctgg cgcagcagga acgaactgaa gagcaactgg caaacatcgc gcgcggtggt 1620
tatgtgctga aagactgcgc cggtcagccg gaactgattt tcatcgctac cggttcagaa 1680
gttgaactgg ctgttgctgc ctacgaaaaa ctgactgccg aaggcgtgaa agcgcgcgtg 1740
gtgtccatgc cgtctaccga cgcatttgac aagcaggatg ctgcttaccg tgaatccgta 1800

ctgccgaaag cggttactgc acgcgttgct gtagaagcgg gtattgctga ctactggtac 1860
aagtatgttg gcctgaacgg tgctatcgtc ggtatgacca ccttcggtga atctgctccg 1920
gcagagctgc tgtttgaaga gttcggcttc actgttgata acgttgttgc gaaagcaaaa 1980
gaactgctgt aa 1992

The present invention also provides a kind of recombinant expression vector containing the encoding gene of a high-stereoselectivity *R* transketolase mutant.

The present invention also provides a kind of host cells with the recombinant expression vector containing the encoding gene of a high-stereoselectivity *R* transketolase mutant.

Further, the high-stereoselectivity *R* transketolase mutant, or the DNA molecules coding the mutant, or recombinant expression plasmids containing DNA molecules for coding the mutant, or the host cells containing the aforementioned recombinant expression plasmids can be used for the synthesis of *R*-aromatic dihydroxyketone.

The present invention also provides a method for preparing the high-stereoselectivity *R* transketolase mutant, which includes the following steps:
Step 1: With the gene derived from prokaryotic E. coli transketolase as a template, obtain the high-stereoselectivity *R* transketolase mutant DNA molecules through PCR site-directed mutation and saturation mutation.
Step 2: Construct a recombinant expression vector containing the high-stereoselectivity *R* transketolase mutant DNA molecules obtained according to the Step 1.
Step 3: Through IPTG induction, produce and obtain a large amount of high-stereoselectivity *R* transketolase mutant with biological activity from the host cells containing the recombinant expression vector obtained according to the Step 2.
Step 4: Obtain high activity and stereoselectivity *R* transketolase mutant protein through affinity chromatography by separating and purifying the high-stereoselectivity *R* transketolase mutant protein with biological activity obtained according to the Step 3.

Further, the DNA molecules mentioned in Step 1 can be obtained through PCR site-directed mutation and saturation mutation by taking the prokaryotic E. coli transketolase gene as a template.

Further, in Step 1, the recombinant plasmid pET28a-TK is used as a template.

Further, double primer pairs are used for PCR site-directed mutation, as represented by D469T-F, D469T-R, R520Q-F, R520Q-R, S385Y-F, and S385Y-R. The nucleotide sequences of D469T-F, D469T-R, R520Q-F, R520Q-R, S385Y-F, and S385Y-R are those represented by SEQ ID NO: 4, SEQ ID NO: 5, SEQ ID NO: 6, SEQ ID NO: 7, SEQ ID NO: 8 and SEQ ID NO: 9, respectively. The double primer pairs as represented by H26-F, H26-R, F434-F, F434-R, L466-F, and L466-R, are used for PCR saturation mutation. The nucleotide sequences of H26-F, H26-R, F434-F, F434-R, L466-F, and L466-R are those represented by SEQ ID NO: 10, SEQ ID NO: 11, SEQ ID NO: 12, SEQ ID NO: 13, SEQ ID NO: 14, and SEQ ID NO: 15, respectively.

Further, the transketolase mutant is generated by the amino acid sequence of the prokaryotic E. coli transketolase through the site-directed mutation and iterative saturation mutation. The serine at Site 385 of the amino acid sequence of the prokaryotic E. coli transketolase is mutated into tyrosine. The aspartic acid at Site 469 is mutated into threonine. The arginine at Site 520 is mutated into glutamine (namely, obtained from the sequence represented by SEQ ID NO: 16). The histidine at Site 26 is mutated into tyrosine. The phenylalanine at Site 434 is mutated into tyrosine. The leucine at Site 466 is mutated into histidine.

Further, In Step 4, a high activity and stereoselectivity *R* transketolase mutant EcTK1_YYH is obtained.

Further, the result shows that the stereoselectivity of the p-methyl sulfonyl phenyl dihydroxyketone synthesized from the p-methyl sulfonyl benzaldehyde under the catalysis of the *Ec*TK1_YYH mutant enzyme is 95.2% *ee* (*R*).

The present invention also provides the application of the high-stereoselectivity *R* transketolase mutant in catalyzing the formation of the high-stereoselectivity *R*-aromatic dihydroxyketone from aromatic aldehyde.

Further, with the p-methyl sulfonyl benzaldehyde as the substrate and the high-stereoselectivity *R* transketolase mutant as the catalytic enzyme, the *R*-p-methyl sulfonyl phenyl dihydroxyketone can be synthesized from p-methyl sulfonyl benzaldehyde.

The present invention also provides the application of the encoding gene of the high-stereoselectivity R transketolase mutant in catalyzing the formation of the high-stereoselectivity *R*-aromatic dihydroxyketones from aromatic aldehyde.

Further, a recombinant expression vector containing the encoding gene of the high-stereoselectivity R transketolase mutant can be constructed. The host cells containing the recombinant expression vector can produce a large amount of high-stereoselectivity *R* transketolase mutant with biological activity through IPTG induction. The high-stereoselectivity R transketolase mutant protein with biological activity can be separated and purified through affinity chromatography to obtain the high activity and stereoselectivity *R* transketolase mutant protein. With the p-methyl sulfonyl benzaldehyde as the substrate and the high activity and stereoselectivity *R* transketolase mutant protein as the catalytic enzyme, the p-methyl sulfonyl benzaldehyde can be synthesized into the *R*-p-methyl sulfonyl phenyl dihydroxyketone.

In the preferred Embodiment 1 of the present invention, the site-directed mutation and saturation mutation of natural transketolase from prokaryotic E. coli and the screening of high activity and high stereoselectivity *R* transketolase are detailed.

In another preferred Embodiment 2 of the present invention, the expression and purification process of the high-stereoselectivity *R* transketolase mutant is detailed.

In another preferred Embodiment 3 of the present invention, the detection process of the high-stereoselectivity product obtained from the p-methyl sulfonyl benzaldehyde under the catalysis of the high-stereoselectivity *R* transketolase mutant EcTK1is detailed.

The present invention modifies the amino acid sequence of the natural transketolase from prokaryotic E. coli based on the technology of site-directed mutation and iterative saturation mutation and obtains the high activity and stereoselectivity *R* transketolase mutant *Ec*TK1_YYH. The stereoselectivity of its catalyzed p-methyl sulfonyl phenyl dihydroxyketone is 95.2% *ee* (*R*). Its beneficial technical effects are: 1. The high activity of the *R* transketolase mutant. 2. The *R*-aromatic dihydroxyketone produced by catalyzing the aromatic aldehyde substrate. 3. High stereoselectivity of the *R*-aromatic dihydroxyketone. 4. The *R*-aromatic dihydroxyketone is an essential chiral drug intermediate, and the *R* transketolase mutant can be used for the biosynthesis of chiral drug intermediates. The mutant obtained by the present invention lays a foundation for its application in the synthesis of chiral *R*-aromatic dihydroxyketones. At present, this is the first research report on the efficient catalysis of aromatic aldehyde to form high-stereoselectivity *R*-aromatic dihydroxyketone by transketolase.

The following will further explain the concept, detailed structure, and technical effects of the present invention in conjunction with the attached figures to understand the purpose, features, and effects fully.

### Brief Description of the Figures

Fig. 1 shows a schematic of the transketo reaction of p-methyl sulfonyl benzaldehyde catalyzed by high-stereoselectivity *R* transketolase in the preferred Embodiment 3 of the present invention.
Fig. 2 shows a reaction schematic of the ammonia-alcohol diastereoisomer transformed by the high-stereoselectivity product obtained from p-methyl sulfonyl benzaldehyde under the catalysis of the high-stereoselectivity *R* transketolase in the preferred Embodiment 3 of the invention.
Fig. 3 shows the liquid phase of the reaction products obtained by taking p-methyl sulfonyl benzaldehyde as the substrate under C18 column conditions.
Fig. 4 shows the liquid phase of the threo reaction products obtained by taking p-methyl sulfonyl benzaldehyde as the substrate under chiral liquid phase conditions.
Fig. 5 shows the H-NMR of the reaction product (1R, 2R)-AMPP.
Fig. 6 shows the C-NMR of the reaction product (1R, 2R)-AMPP.
Fig. 7 shows the liquid phase of the reaction products obtained by taking benzaldehyde as the substrate under C18 column conditions, i.e., the high-performance liquid chromatography identification of the benzaldehyde reaction products obtained through enzyme cascade catalysis. Among them, A is the HPLC chromatography of the enzyme cascade reaction products, B is the standard (1R, 2R)-phenylserinol and C is the standard mixture of threo and erythro products.
Fig. 8 shows the liquid phase of the reaction products obtained by taking p-methyl benzaldehyde as the substrate under C18 column conditions, i.e., the high-performance liquid chromatography identification of the p-methyl benzaldehyde reaction products obtained through enzyme cascade catalysis. Among them, A is the HPLC chromatography of the enzyme cascade reaction products, B is the standard (1R, 2R)-p-methyl phenylserinol and C is the standard mixture of threo and erythro products.

### Detailed Description of the Preferred Embodiments

The following introduces preferred Embodiments of the present invention based on the attached specification figures for a clearer and easier understanding of its technology. The present invention can be shown through different forms of Embodiments. The present invention's protection scope is not limited to the Embodiments stated in the Specification.

### Embodiment 1: Site-directed mutation, saturation mutation and screening

Natural transketolase from prokaryotic E. coli was subjected to site-directed mutation and saturation mutation, and high activity and stereoselectivity R transketolase was found after the screening. The primer nucleotide sequences are shown in Table 1, where n refers to any base within a, t, c, and g, and k refers to base t or g. The degenerate codon nnk can be used for coding 20 amino acids at random. M refers to base a or c, and mnn and nnk complement each other.

**Table 1 Primer nucleotide sequences**

| Primer name | Nucleotide sequence | SEQ ID |
|---|---|---|
| D469T-F | tccatcggtctgggcgaaaccggcccgactcaccagccggttga | NO: 4 |
| D469T-R | ggctggtgagtcgggccggtttcgcccagaccgatggagtcgtg | NO: 5 |
| R520Q-F | accgcactgatcctctcccaacagaacctggcgcagcaggaacgaa | NO: 6 |
| R520Q-R | ctgctgcgccaggttctgttgggagaggatcagtgcggtcgggccg | NO: 7 |
| S385Y-F | tctgctgacctggcgccgtataacctgaccctgtggtctggttcta | NO: 8 |
| S385Y-R | agaccacagggtcaggttatacggcgccaggtcagcagaaccgccg | NO: 9 |
| H26-F | aagccaaatccggt**nnk**ccgggtgcccctatgggtat | NO: 10 |
| H26-R | ataggggcacccgg**mnn**accggatttggctttctgta | NO: 11 |
| F434-F | cgtacacctccacc**nnk**ctgatgttcgtggaatacgc | NO: 12 |
| F434-R | tccacgaacatcag**mnn**ggtggaggtgtacggcagga | NO: 13 |
| L466-F | acgactccatcggt**nnk**ggcgaaaccggcccgactca | NO: 14 |
| L466-R | geeccggtttcgcc**mnn**accgatggagtcgtgggtgt | NO: 15 |

(1) With the recombinant plasmid pET28a-TK as the template, its nucleotide sequence is represented by SEQ ID NO: 3. With D469T-F and D469T-R as the primers, using the Phanta Max polymerase (purchased from Vazyme Biotech) for PCR amplification (95°C for 5min. 95°C for 30s. 65°C for 30 s. 72°C for 7.5min. 34 cycles. 72°C for 10min). Transform the PCR product directly into E. coli BL21 (DE3) receptive cells after FD-Dpn I digestion (incubator at 30°C, 6h). After the resuscitation fluid has been blown and suctioned thorough equal mixing, add roughly 1/8 of the resuscitation fluid to a kanamycin-resistant LB plate. Finally, culture it at 37°C for 12-16h.
   SEQ ID NO: 3: 
tggcgaatgg gacgcgccct gtagcggcgc attaagcgcg gcgggtgtgg tggttacgcg 60
cagcgtgacc gctacacttg ccagcgccct agcgcccgct cctttcgctt tcttcccttc 120
ctttctcgcc acgttcgccg gctttccccg tcaagctcta aatcgggggc tccctttagg 180
gttccgattt agtgctttac ggcacctcga ccccaaaaaa cttgattagg gtgatggttc 240
acgtagtggg ccatcgccct gatagacggt ttttcgccct ttgacgttgg agtccacgtt 300
ctttaatagt ggactcttgt tccaaactgg aacaacactc aaccctatct cggtctattc 360
ttttgattta taagggattt tgccgatttc ggcctattgg ttaaaaaatg agctgattta 420
acaaaaattt aacgcgaatt ttaacaaaat attaacgttt acaatttcag gtggcacttt 480
tcggggaaat gtgcgcggaa cccctatttg tttatttttc taaatacatt caaatatgta 540
tccgctcatg aattaattct tagaaaaact catcgagcat caaatgaaac tgcaatttat 600
tcatatcagg attatcaata ccatattttt gaaaaagccg tttctgtaat gaaggagaaa 660
actcaccgag gcagttccat aggatggcaa gatcctggta tcggtctgcg attccgactc 720
gtccaacatc aatacaacct attaatttcc cctcgtcaaa aataaggtta tcaagtgaga 780
aatcaccatg agtgacgact gaatccggtg agaatggcaa aagtttatgc atttctttcc 840
agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
 
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700
ggtcactgat gcctccgtgt aagggggatt tctgttcatg ggggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatatcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
 
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatctc gatcccgcga 4980
aattaatacg actcactata ggggaattgt gagcggataa caattcccct ctagaaataa 5040
ttttgtttaa ctttaagaag gagatatacc atgggcagca gccatcatca tcatcatcac 5100
agcagcggcc tggtgccgcg cggcagccat atgatgtcct cacgtaaaga gcttgccaat 5160
gctattcgtg cgctgagcat ggacgcagta cagaaagcca aatccggtca cccgggtgcc 5220
cctatgggta tggctgacat tgccgaagtc ctgtggcgtg atttcctgaa acacaacccg 5280
cagaatccgt cctgggctga ccgtgaccgc ttcgtgctgt ccaacggcca cggctccatg 5340
ctgatctaca gcctgctgca cctcaccggt tacgatctgc cgatggaaga actgaaaaac 5400
ttccgtcagc tgcactctaa aactccgggt cacccggaag tgggttacac cgctggtgtg 5460
gaaaccacca ccggtccgct gggtcagggt attgccaacg cagtcggtat ggcgattgca 5520
gaaaaaacgc tggcggcgca gtttaaccgt ccgggccacg acattgtcga ccactacacc 5580
tacgccttca tgggcgacgg ctgcatgatg gaaggcatct cccacgaagt ttgctctctg 5640
gcgggtacgc tgaagctggg taaactgatt gcattctacg atgacaacgg tatttctatc 5700
gatggtcacg ttgaaggctg gttcaccgac gacaccgcaa tgcgtttcga agcttacggc 5760
tggcacgtta ttcgcgacat cgacggtcat gacgcggcat ctatcaaacg cgcagtagaa 5820
gaagcgcgcg cagtgactga caaaccttcc ctgctgatgt gcaaaaccat catcggtttc 5880
ggttccccga acaaagccgg tacccacgac tcccacggtg cgccgctggg cgacgctgaa 5940
attgccctga cccgcgaaca actgggctgg aaatatgcgc cgttcgaaat cccgtctgaa 6000
atctatgctc agtgggatgc gaaagaagca ggccaggcga aagaatccgc atggaacgag 6060
aaattcgctg cttacgcgaa agcttatccg caggaagccg ctgaatttac ccgccgtatg 6120
aaaggcgaaa tgccgtctga cttcgacgct aaagcgaaag agttcatcgc taaactgcag 6180
gctaatccgg cgaaaatcgc cagccgtaaa gcgtctcaga atgctatcga agcgttcggt 6240
ccgctgttgc cggaattcct cggcggttct gctgacctgg cgccgtctaa cctgaccctg 6300
tggtctggtt ctaaagcaat caacgaagat gctgcgggta actacatcca ctacggtgtt 6360
cgcgagttcg gtatgaccgc gattgctaac ggtatctccc tgcacggtgg cttcctgccg 6420
tacacctcca ccttcctgat gttcgtggaa tacgcacgta acgccgtacg tatggctgcg 6480
ctgatgaaac agcgtcaggt gatggtttac acccacgact ccatcggtct gggcgaagac 6540
ggcccgactc accagccggt tgagcaggtc gcttctctgc gcgtaacccc gaacatgtct 6600
acatggcgtc cgtgtgacca ggttgaatcc gcggtcgcgt ggaaatacgg tgttgagcgt 6660
caggacggcc cgaccgcact gatcctctcc cgtcagaacc tggcgcagca ggaacgaact 6720
gaagagcaac tggcaaacat cgcgcgcggt ggttatgtgc tgaaagactg cgccggtcag 6780
ccggaactga ttttcatcgc taccggttca gaagttgaac tggctgttgc tgcctacgaa 6840
 
aaactgactg ccgaaggcgt gaaagcgcgc gtggtgtcca tgccgtctac cgacgcattt 6900
gacaagcagg atgctgctta ccgtgaatcc gtactgccga aagcggttac tgcacgcgtt 6960
gctgtagaag cgggtattgc tgactactgg tacaagtatg ttggcctgaa cggtgctatc 7020
gtcggtatga ccaccttcgg tgaatctgct ccggcagagc tgctgtttga agagttcggc 7080
ttcactgttg ataacgttgt tgcgaaagca aaagaactgc tgtaactcga gcaccaccac 7140
caccaccact gagatccggc tgctaacaaa gcccgaaagg aagctgagtt ggctgctgcc 7200
accgctgagc aataactagc ataacccctt ggggcctcta aacgggtctt gaggggtttt 7260
ttgctgaaag gaggaactat atccggat 7288
(2) Select three single colonies from the dilution mentioned above plate and culture them in kanamycin resistant LB medium at 37°C for 7-8h. Use part of the medium for sequencing and temporarily put the other part in a 4°C refrigerator for short-term storage. Transfer the sequenced positive transformant medium to a fresh SmL LB medium with kanamycin resistance at a 1% inoculation amount. Perform an overnight culture, and extract the plasmid. Take the plasmid (pET28a-TK/D469T) as a template and R520Q-F and R520Q-R as primers to perform a whole-plasmid amplification. Repeat the above plasmid construction method. Then extract the pET28a TK/D469T/R520Q plasmid and take it as a template with S385Y-F and S385Y-R as primers to perform a whole-plasmid amplification. Repeat the above plasmid construction method to construct the pET28a-TK/D469T/R520Q/S385Y (pET28a- *Ec*TK1) plasmid. Take the pET28a-*Ec*TK1 plasmid as a template with H26-F and H26-R as the primers to perform a whole-plasmid amplification. Repeat the above method to obtain a recombinant sub library.
(3) Select all the single colonies (about 40) from the dilution mentioned above the plate and culture them in kanamycin-resistant LB medium at 37°C for 7-8h. Use part of the medium for sequencing, and temporarily put the other part in a 4°C refrigerator for short-term storage. Transfer the sequenced positive transformant medium to a 24-hole deep hole plate containing fresh SmL LB medium with kanamycin resistance at 1% inoculation amount. After 3h of cultivation at 37 °C, add 0.4mM IPTG inducer to induce efficient expression of the recombinant gene at 30°C for 5h. Centrifuge the recombinant cells at 4000rpm to collect thalli and store them at -80°C. Use the same induction method to obtain recombinant thallus pET28a EcTK1 for control.
(4) Add the obtained recombinant thalli to the 300µl 50mM Tris-Cl (pH=7.5) for suspension and ultrasonic fragmentation, and centrifuge it at a low temperature to get the supernatant. Add 20% v/v supernatant into the reaction liquid containing 5mM p-methyl sulfonyl benzaldehyde and 30µM R transaminase for the reaction for 6h at 30°C. Since the whole thalli contains ketol isomerase that can accelerate the racemization of dihydroxyketone, to accurately examine the stereoselectivity of the dihydroxyketone, couple a stereospecific R-transaminase, transiently transform the dihydroxyketone into a stable chiral amino diol compound. Examine the dihydroxyketone's chirality by detecting the amino diol's chirality.
(5) Detect the reaction product described in (4) through liquid chromatography to examine the ratio of (1S, 2R)-amino diol to (1R, 2R)-amino diol. Liquid phase analysis conditions: Chromatographic column super-C18 (250mm × 4.6mm, particle size 5µm). Phase A: H₂O (containing 10mM KH₂PO₄, pH=8.5), Phase B: acetonitrile, chromatographic conditions: 0min 98:2 to 20min 90:10 linear change, 1ml/min, 224nm, retention time results for (1S, 2R)-amino diol and (1R, 2R)-amino diol are 9.7 and 10.9 min, respectively. The result shows that the R-stereoselectivity of *Ec*TK1_Y is the highest for saturated mutants. And its activity is also high so that *Ec*TK1_Y can be selected as the template for the next round of saturation mutation with F434-F/R as the primer for whole-plasmid PCR to repeat the plasmid construction, expression, and detection methods mentioned above. The R-stereoselectivity of *Ec*TK1_YY is the highest for the second round of saturated mutants. As a result, it can be used as the template for the third round of saturation mutation with F434-F/R as the primer to repeat the steps mentioned above to obtain the *Ec*TK1_YYH mutant finally.

### Embodiment 2: Expression and purification of the transketolase mutant

Expression and purification method of Mutant EcTK1 _YYH: Select single colonies of *E. coli* BL21 (DE3) containing recombinant plasmids from the LB solid medium and inoculate it to a 40 ml LB liquid medium (with 50µg/ml kanamycin antibiotic). Perform an overnight culture at 37°C and 220rpm. Then Transfer 7.5ml of bacterial culture medium to 2L shake flasks containing 500 ml of LB liquid medium, inoculate it in two bottles, perform a culture at 37°C until the OD600 reaches 0.6-0.8, add 0.4mM IPTG for induction, and induce the culture at 30 °C and 200 rpm for 5h. Collect 1L of fermentation broth and centrifuge it at 5000 rpm for 20min to collect cells. Resuspend the collected cells in a 30mL nickel column-binding buffer and place them in an ice-water mixture for ultrasonic fragmentation. Ultrasonic fragmentation conditions: Operating for 5s and then pausing for 10s. Total time: 30 min. Centrifuge the mixture after fragmentation at 12000 rpm for 1h, and filter the supernatant through 0.22µm membrane filtration. Then load the filtered sample with 2ml of nickel filler pre-balanced with nickel column binding buffer. Wash the heteroproteins with 50 mM imidazole elution buffer (10 times the column volume), and then elute the target protein with 5 ml of 250 mM imidazole elution buffer. Collect samples with different tubes (500µl/tube). Determine the protein concentration of each tube with a micro ultraviolet-visible spectrophotometer (NanoDrop), combine several tubes of protein solution with higher concentration, dilute or concentrate it to 2.5 ml, and load the desalting column sample balanced by glycerol buffer. After draining the protein solution, add 3.5mL glycerol buffer to elute the protein.

### Embodiment 3: Examining the stereoselectivity of the product obtained from p-methyl sulfonyl benzaldehyde catalyzed by EcTK1 mutant

The high-stereoselectivity product detection process obtained from p-methyl sulfonyl benzaldehyde catalyzed by the high-stereoselectivity R transketolase mutant EcTK1 involves chemical reactions, including transketo and transamination.

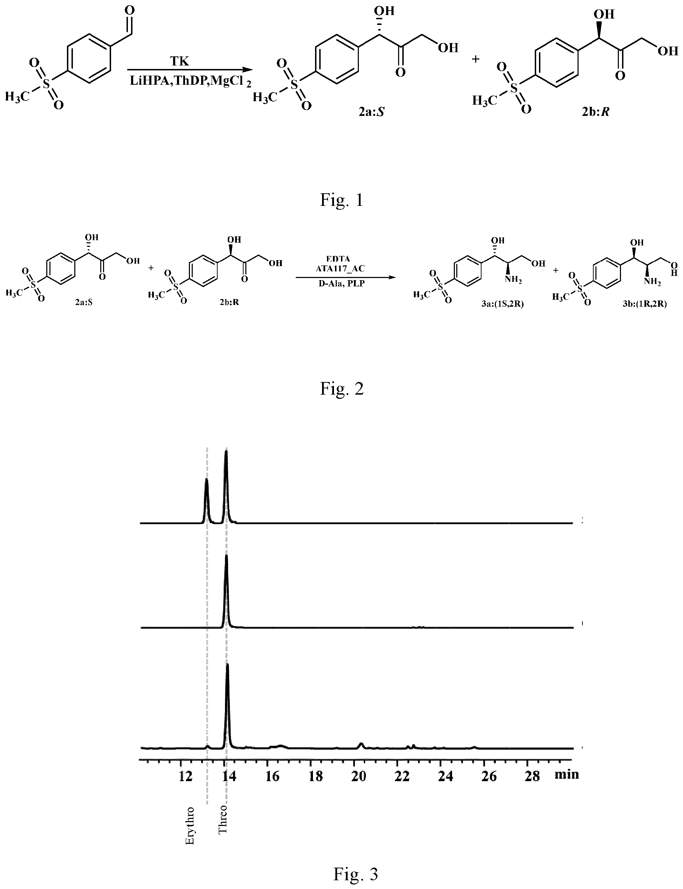

As shown in Fig. 1, the principle of the transketo reaction is based on taking the p-methyl sulfonyl benzaldehyde as the substrate and the transketolase mutant as the catalyst. The catalyst is expressed by "TK." The generation of S- and R-dihydroxyketone products is possible in the presence of LiHPA, MgCl2, and ThDP. The S-dihydroxyketone is expressed by "**2a**: S", and the *R*-dihydroxyketone product is expressed by "**2b**: *R".*

As shown in Fig. 2, the principle of the transamination reaction is to transform the transamination reaction product, dihydroxyketone into a stable chiral amino diol compound instantly. The stereoselectivity of dihydroxyketone is examined by detecting the stereoselectivity of the amino diol. The transketo reaction products, S-dihydroxyketone and *R*-dihydroxyketone, can be transformed into (1S, 2R) - ammonia-alcohol and (1R, 2R) - ammonia-alcohol, respectively, under the action of D-Ala, PLP, NADH, LDH, and transaminase ATA117 _AC. The (1S, 2R) - ammonia-alcohol is expressed by "**3a**: (1S, 2*R*)", and (1R, 2R) - ammonia-alcohol is expressed by "**3b**: (1R, 2R)". The concentrations of the (1S, 2R) - ammonia-alcohol and (1R, 2R) - ammonia-alcohol obtained through liquid phase detection can be used to characterize the concentrations of transketo products **2a** and **2b**, respectively.
(1) The volume of the reaction system is 100µl (50mM Tris-Cl buffer, pH 7) in total, including 5mM p-methyl sulfonyl benzaldehyde, 25mM LiHPA, 9mM MgCl₂, 4.8mM ThDP, 100µM pure transketolase mutant protein. Perform a reaction at 25 °C for 20min, and then terminate the transketo reaction by adding 18mM EDTA chelating Mg²⁺. Suck and add 20µl transketo reaction liquid to a new 100µl system, add 200mM D-Ala, 2mM PLP, 20mM NADH, 90U/ml LDH, and 200µM transaminase ATA117 _AC for reaction at 25 °C for 30min. Therefore, the *S*- and *R*-hydroxyketone products can completely transform into the (1S, 2R) - and (1R, 2R) - ammonia-alcohol diastereoisomers. The concentration of hydroxyketone enantiomers can be examined by detecting the concentration of ammonia-alcohol diastereoisomers.
(2) The ammonia-alcohol products are analyzed through Agilent liquid chromatography. Chromatographic column super C18 (4.6 × 250 mm, particle size 5µm). Phase A: H₂O (containing 10 mM KH2PO4, pH=8.5), Phase B: Acetonitrile, 1ml/min, and 224nm. The retention time results for (1S, 2R) - and (1R, 2R) - ammonia-alcohols are 9.7 and 10.9 minutes, respectively. The chromatographic conditions are shown in Table 2:

**Table 2 Chromatographic conditions**

| Time | Mobile phase Phase A: Phase B |
|---|---|
| 0min | 98:2 |
| 15min | 92:8 |
| 17min | 70:30 |
| 21min | 70:30 |
| 22min | 98:2 |
| 27min | 98:2 |

(3) With E. coli *Ec*TK1 as the female parent, in the three rounds of iterative saturation mutation, the key mutants can be used to catalyze 5 mM p-methyl sulfonyl benzaldehyde substrate respectively at 25 °C for 20 min, and the transformation ratio C (%) and stereoselectivity *ee* (%) results of the produced *R*-hydroxyketone are shown in Table 3.

**Table 3 Transformation ratio C (%) and stereoselectivity ee (%) of R-hydroxyketone**

| TK | C(%) | *ee* (%) |
|---|---|---|
| *Ec*TK1_ | 87.0±1.5 | 93.3±1.1(*S*) |
| *Ec*TK1_Y (H26Y) | 82.5±0.4 | 4.8±0.0 (*R*) |
| *Ec*TK1_YY (H26Y+F434Y) | 85.3±0.7 | 90.8±0.1 (*R*) |
| *Ec*TK1_YYF (H26Y+F434Y+L466F) | 92.9±0.1 | 91.6±0.1(*R*) |
| *Ec*TK1_YYH (H26Y+F434Y+L466H) | 87.2±0.4 | 95.2±0.1(*R*) |

### Embodiment 4: Application of the EcTK1 mutant to the synthesis of (1R, 2R)-AMPP and its derivatives

The synthesis method of (1R, 2R)-AMPP ((1R, 2R) -2-amido-1-(4-(mesyl) phenyl) propane -1,3-diol, also known as (1R, 2R) -1,3-dihydroxy-2-amido-1-p-methyl sulfonyl phenyl propane) and its derivatives. With the benzaldehyde derivative (Formula 1) as the substrate, through the transketo reaction under the action of E. coli transketolase mutant, the reaction products shown in Formula 2 can be obtained. Then with the compound shown in Formula 2 as the substrate, the transamination reaction is carried out under the action of the transaminase ATA117 mutant. The amino donors are D-alanine, D-glycine, D-valine, D-leucine, D-isoleucine D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine, the (1R, 2R)-AMPP and its derivatives shown in Formula 3 can be obtained through their synthesis.

The reaction formulas involved are as follows:

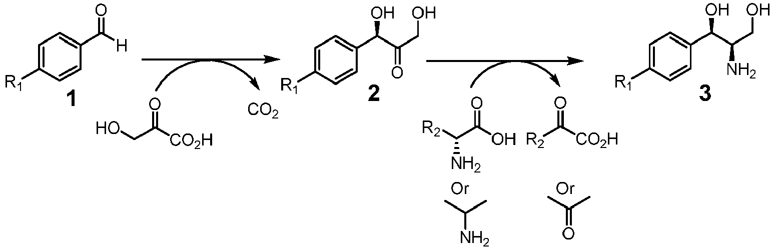

The following is a detailed explanation of this method:

### Step 1: Expression and purification of E. coli transketolase mutant

Introduce the nucleotide sequence with the above-mentioned amino acid mutation in the sequence represented by SEQ ID NO: 17 into Vector pET28a and host E.coli BL21. Then select the single colony of E. coli BL21 containing recombinant plasmids from the LB solid medium and inoculate them to a 40 ml LB liquid medium (with 50µg/ml kanamycin antibiotic), perform an overnight culture at 37°C and 220rpm. Transfer 10 ml of bacterial culture medium to 2L shake flasks containing 500 ml of LB liquid medium, inoculate it in two bottles, perform a culture at 37°C and 220 rpm until the OD600 reaches 0.6-0.8, add 0.2mM IPTG for induction, and induce the culture at 30 °C and 200 rpm for 5h.

After the cultivation, collect 1L of fermentation broth and centrifuge it at 5000 rpm for 20min to collect cells. Resuspend the collected cells in a 30 mL nickel column-binding buffer and place them in an ice-water mixture for ultrasonic fragmentation. Ultrasonic fragmentation conditions: Operating for 5s and then pausing for 10s. Total time: 30 min.

Centrifuge the mixture after fragmentation at 12000 rpm for 30min, and filter the supernatant through 0.22µm membrane filtration. Then load the filtered sample with 2ml of nickel filler pre-balanced with nickel column binding buffer. Wash the heteroproteins with 50 mM imidazole elution buffer (20 times the column volume), and then elute the target protein with 5 ml of 250 mM imidazole elution buffer. Collect samples with different tubes (500µl/tube). Determine the protein concentration of each tube, combine several tubes of protein solution with higher concentration, dilute or concentrate it to 2.5 ml, and load the desalting column sample balanced by glycerol buffer. After the protein solution is drained, add 3.5ml glycerol buffer to elute the protein. Thus, it is possible to obtain the transketolase mutant E. coli pure protein.

In Step 1, the protein concentration can be measured using a Thermo Scientific Nanodrop 8000 detector to detect the absorbance value E at 280nm. The target protein concentration can be converted according to the molar extinction coefficient, i.e., the protein concentration (mg/mL)=E/molar extinction coefficient. The program Vector NTI can forecast the recombinase's molar extinction coefficient.

### Step 2: Expression and purification of transaminase ATA117 mutant

Introduce the nucleotide sequence with the above-mentioned amino acid mutation in the sequence represented by SEQ ID NO: 19 into Vector pRSFDuet and host E.coli BL21. Then select the single colony of E. coli BL21 containing recombinant plasmids from the LB solid medium and inoculate it to a 40 ml LB liquid medium (with 50µg/ml kanamycin antibiotic), perform an overnight culture at 37°C and 220rpm. Transfer 10 ml of bacterial culture medium to 2L shake flasks containing 500 ml of LB liquid medium, inoculate it in two bottles, perform a culture at 37°C and 220 rpm until the OD600 reaches 0.6-0.8, add 0.2 mM IPTG for induction, and induce the culture at 20°C and 200 rpm for 15h.

After the cultivation, collect 1L of fermentation broth and centrifuge it at 5000 rpm for 20min to collect cells. Resuspend the collected cells in a 30 mL nickel column-binding buffer and place them in an ice-water mixture for ultrasonic fragmentation. Ultrasonic fragmentation conditions: Operating for 5s and then pausing for 10s. Total time: 30 min.

Centrifuge the mixture after fragmentation at 12000 rpm for 30min, and filter the supernatant through 0.22µm membrane filtration. Then load the filtered sample with 2ml of nickel filler pre-balanced with nickel column binding buffer. Wash the heteroproteins with 50 mM imidazole elution buffer (10 times the column volume), and then elute the target protein with 5 ml of 250 mM imidazole elution buffer. Collect samples with different tubes (500µl/tube). Since the transaminase binds cofactor PLP, it is yellow. Combine several dark yellow protein solution tubes, dilute or concentrate the protein solution to 2.5 ml, and load the chromatographic column sample balanced by glycerol buffer. After the protein solution is drained, add 3.5ml glycerol buffer to elute the protein, thus, the pure protein of the transaminase can be obtained.

### Step 3: Synthesis of (1R, 2R)-AMPP and its derivatives

Add 10mM benzaldehyde derivatives, 30mM lithium hydroxypyruvate (LiHPA, as a transketotic donor), 4.8mM thiamine pyrophosphate (TPP, as the cofactor for the transketo reaction), 18 mM MgCl₂ (as the metal ions for the transketotic reaction), and 100µM E. coli transketolase mutant in a 50µl Tris-HCl buffer containing 100mM (pH 7.5) for the reaction for 1-3h at 25°C.

Add 100mM Tris-HCl buffer (pH 7.5), 200mM D-Ala, or D-glycine, or D-valine, or D-leucine, or D-isoleucine, or D-methionine, or D-proline, or D-tryptophan, or D-serine, or D-tyrosine, or D-cysteine, or D-phenylalanine, or D-asparagine, or D-glutamine, or D-threonine, or D-aspartic acid, or D-glutamic acid, or D-lysine, or D-arginine, or D-histidine or isopropylamine (as a transamination donor), 2 mM pyridoxal phosphate (PLP, as the cofactor for the transamination reaction), 50µM transaminase ATA117 mutant, expand the reaction system to 100µl. The (1R, 2R) - AMPP and its derivatives can be obtained after the reaction for 3-6h at 25°C.

**Table 4 Transformation ratio and stereoselectivity of the cascade reaction products**

| Embodiment | Substrate | Amino donor | E. coli transketolase | Transaminase | Transformation ratio | *de* | *ee* |
|---|---|---|---|---|---|---|---|
| 1 | P-methyl sulfonyl benzaldehyde | D-alanine | H26Y | V69A+F122C+I157 H | *** | * | ***** |
| 2 | P-methyl sulfonyl benzaldehyde | D-alanine | H26Y+F434Y | V69A+F122C+I157 H | *** | *** | ***** |
| 3 | P-methyl sulfonyl benzaldehyde | D-alanine | H26Y+F434Y+L46 6H | V69A+F122C | *** | *** | ***** |
| 4 | P-methyl sulfonyl benzaldehyde | D-alanine | H26Y+F434Y+L46 6H | V69A+F122C+I157 H | *** | **** | ***** |
| 5 | P-methyl sulfonyl benzaldehyde | D-alanine | H26Y+F434Y+L46 6H | V69A+F122C+I157 H+F225H | *** | **** | ***** |
| 6 | P-methyl sulfonyl benzaldehyde | Isopropylamine | H26Y+F434Y | V69A+F122C+I157 H | *** | *** | ***** |
| 7 | P-methyl sulfonyl benzaldehyde | Isopropylamine | H26Y+F434Y+L46 6H | V69A+F122C+I157 H | *** | **** | ***** |
| 8 | Benzaldehyde | D-alanine | H26Y+F434Y | V69A+F122C+I157 H | ** | *** | ***** |
| 9 | Benzaldehyde | D-alanine | H26Y+F434Y+L46 6F | V69A+F122C+I157 H+F225H | ** | **** | ***** |
| 10 | Benzaldehyde | sopropylamine | H26Y+F434Y | V69A+F122C+I157 H | ** | *** | ***** |
| 11 | Benzaldehyde | sopropylamine | H26Y+F434Y+L46 6F | V69A+F122C+I157 H | ** | **** | ***** |
| 12 | P-methyl benzaldehyde | D-alanine | H26Y+F434Y | V69A+F122C+I157 H | ** | *** | ***** |
| 13 | P-methyl benzaldehyde | D-alanine | H26Y+F434Y+L46 6F | V69A+F122C+I157 H+F225H | ** | **** | ***** |
| 14 | P-methyl benzaldehyde | Isopropylamine | H26Y+F434Y | V69A+F122C+I157 H | ** | *** | ***** |
| 15 | P-methyl benzaldehyde | Isopropylamine | H26Y+F434Y+L46 6F | V69A+F122C+I157 H | ** | **** | ***** |

| | | | | | | | |
|---|---|---|---|---|---|---|---|
| *: Transformation ratio within 0-40%. **: Transformation ratio within 40-60%. ***: Transformation ratio within 60-80%. ****: Transformation ratio being >80%. **: de* within 0-40%. **: *de* within 40-70%. ***: *de* within 70-90%. ****: *de* being >90%. **: ee* within 0-40%. **: *ee* within 40-70%. ***: *ee* within 70-90%. **** *ee* within 90-99%. *****: *ee* being >99%. | | | | | | | |

Further, the present invention detects the reaction products through an Agilent 1200 liquid chromatograph. Detailed detection method: Using a C18 column (4.6x150mM, particle size 3µm). Column temperature: 30 °C, 0.5 ml/min. Phase A: H₂O (10mM KH₂PO₄, pH=8.5). Phase B: Acetonitrile.

**Table 5 Liquid chromatography conditions**

| | Product | | HPLC method | | Wavelength (nm) | Retention time (min) |
|---|---|---|---|---|---|---|
| P-methyl sulfonyl phenylserinol | | Time | Phase B gradient | | 224 | |
| | | 0min | 2% | | | *Erythro*: 13.4 |
| | | 15min | 10% | | | *Threo*: 14.2 |
| | Phenylserinol | Time | Phase B gradien | | 210 | |
| | | 0min | 5% | | | *Erythro*: 12.5 |
| | | 15min | 20% | | | *Threo*: 13.4 |
| | | 18min | 30% | | | |
| | P-methyl phenylserinol | Time | Phase B gradient | | 220 | |
| | | 0min | 5% | | | *Erythro*: 14.9 |
| | | 15min | 30% | | | *Threo*: 15.6 |
| | | 22min | 30% | | | |

Fig. 3 shows the liquid phase of the reaction products by using p-methyl sulfonyl benzaldehyde as the substrate in Experimental Group 5 under C18 column conditions. In Fig. 3, the upper part shows the liquid phase of (erythro) - p-methyl sulfonyl phenylserinol and (threo) - standard p-methyl sulfonyl phenylserinol. The middle part shows the standard (1R, 2R) - p-methyl sulfonyl phenylserinol. The bottom shows the reaction products obtained according to the Experimental Group 5. As shown in Fig. 3. Threo products are mainly prepared according to the Experimental Group 5. According to the Formula: *de* = [(threo - erythro)/ (threo+erythro)] x 100%, the calculated result is *de* > 90%, where the erythro and threo represent (erythro) - and (threo) - p-methyl sulfonyl phenylserinol.

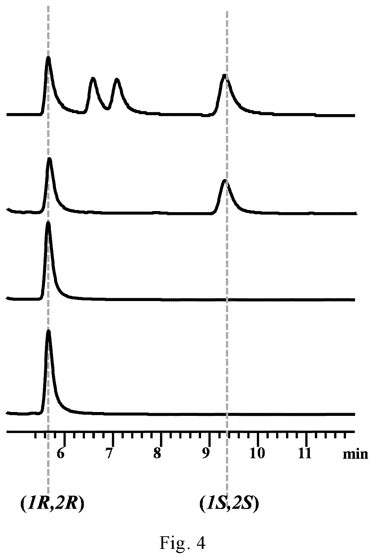

According to the liquid phase peaks, collect the (threo) - p-methylsulfonylphenylserinol product, concentrate it, and then separate it with a chiral liquid phase column to examine the enantioselectivity of the product. Analytical conditions for enantiomers: Chiral column IG, column temperature 25°C, 0.5ml/min, 224nm. Chromatographic conditions: Pure methanol (containing 0.1% diethylamine), 15 min. Fig. 4 shows the liquid phase of the (threo) - p-methyl sulfonyl phenylserinol product under chiral liquid phase column conditions. In Fig. 4, the top shows the control among standard erythro + threo products. The second line shows the control among standard threo products. The third line shows the control among standard (1R, 2R) - p-methyl sulfonyl phenylserinol products. The (threo) - p-methyl sulfonyl phenylserinol product is shown in the fourth and final line. As shown in Fig. 4, the retention time results for (1R, 2R) - and (1S, 2S) - p-methyl sulfonyl phenylserinol are 5.6 and 9.4 min, respectively. According to the Formula *ee* = [(RR-SS)/(RR+SS)] × 100%, the calculated *ee* value is higher than 99%, where RR and SS represent (1R, 2R) - and (1S, 2S) - p-methyl sulfonyl phenylserinol.

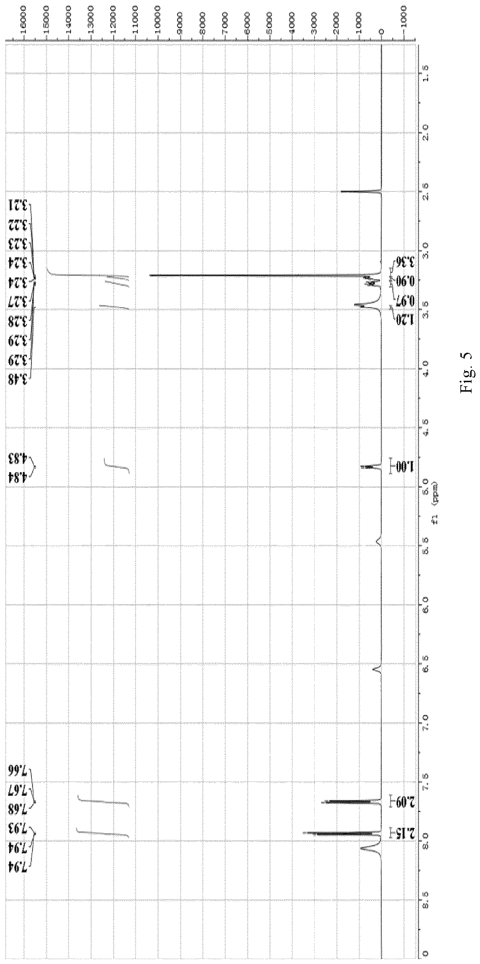

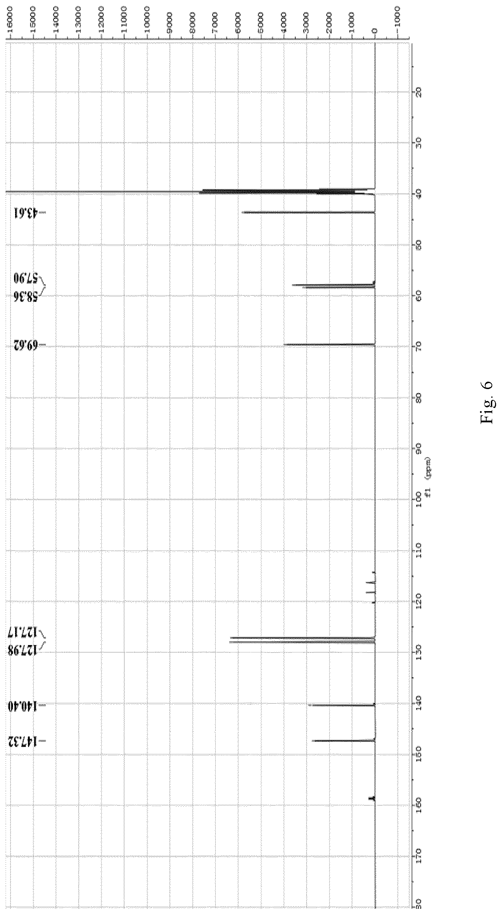

Fig. 5 shows the H-NMR of the reaction product (1R, 2R)-AMPP, and Fig. 6 shows the C-NMR of the reaction product (1R, 2R)-AMPP. According to the standard product control shown in Fig. 5-Fig. 6 and Fig. 3- Fig. 4, the (1R, 2R) - AMPP is synthesized based on the present invention. Considering the *de* and *ee* values, the (1R, 2R)-AMPP prepared according to the method provided by the present invention has a high stereoselectivity.

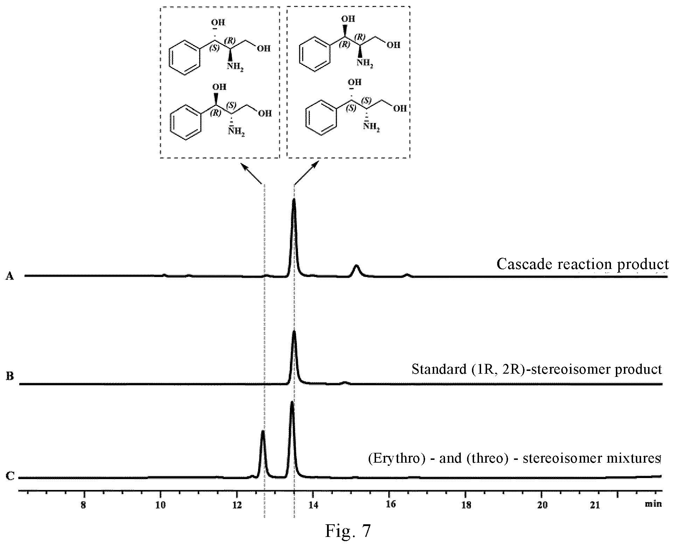

Fig. 7 shows the liquid phase of the reaction product obtained according to Experimental Group 9 by using benzaldehyde as the substrate under C18 column conditions. In Fig. 7, the upper part shows the reaction product obtained according to Experimental Group 9. The middle part shows the standard (1R, 2R) - p-methyl sulfonyl phenylserinol, and the lower part shows the standard (erythro) - phenylserinol and (threo) - phenylserinol products. As shown in Fig. 7, threo products are mainly prepared according to the Experimental Group 9, and according to the Formula: *de* = [(threo - erythro)/(threo + erythro)] x 100%, the calculated result is *de* > 90%, where the erythro and threo represent (erythro) - and (threo) - phenylserinol.

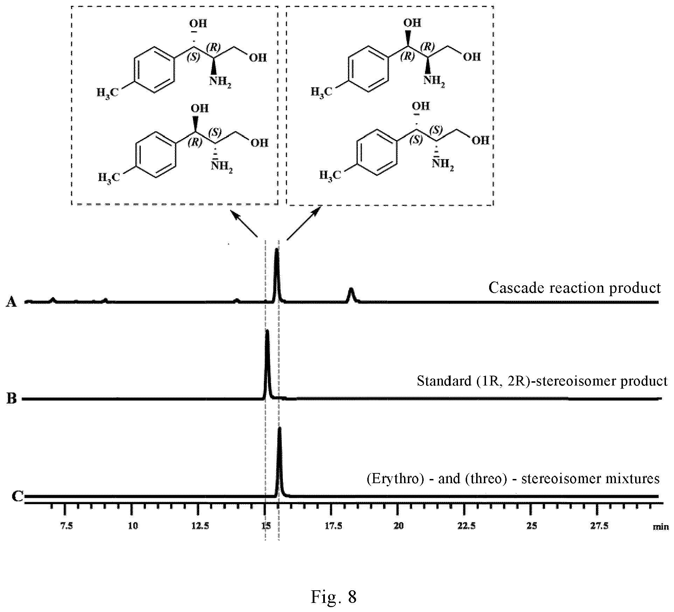

Fig. 8 shows the liquid phase of the reaction product obtained according to the Experimental Group 13 by using p-methyl benzaldehyde as the substrate under C18 column conditions. In Fig. 8, the upper part shows the reaction product obtained according to the Embodiment 13. The middle part shows the (erythro) -p-methyl phenylserinol, and the lower part shows the standard (threo) - p-methyl phenylserinol product. As shown in Fig. 8, threo products are mainly prepared according to the Experimental Group 13, and according to the Formula: *de* = [(threo - erythro)/(threo + erythro)] x 100%, the calculated result is *de >* 90%, where the erythro and threo represent (erythro) - and (threo) - p-methyl phenylserinol.
Amino acid sequences of E. coli transketolase (i.e., E.coli transketolase mutant TK/D469T/R520/S385Y) (SEQ ID NO: 16: 
1 MSSRKELANA IRALSMDAVQ KAKSGHPGAP MGMADIAEVL WRDFLKHNPQ NPSWADRDRF
61 VLSNGHGSML IYSLLHLTGY DLPMEELKNF RQLHSKTPGH PEVGYTAGVE TTTGPLGQGI
121 ANAVGMAIAE KTLAAQFNRP GHDIVDHYTY AFMGDGCMME GISHEVCSLA GTLKLGKLIA
181 FYDDNGISID GHVEGWFTDD TAMRFEAYGW HVIRDIDGHD AASIKRAVEE ARAVTDKPSL
241 LMCKTIIGFG SPNKAGTHDS HGAPLGDAEI ALTREQLGWK YAPFEIPSEI YAQWDAKEAG
301 QAKESAWNEK FAAYAKAYPQ EAAEFTRRMK GEMPSDFDAK AKEFIAKLQA NPAKIASRKA
361 SQNAIEAFGP LLPEFLGGSA DLAPYNLTLW SGSKAINEDA AGNYIHYGVR EFGMTAIANG
421 ISLHGGFLPY TSTFLMFVEY ARNAVRMAAL MKQRQVMVYT HDSIGLGETG PTHQPVEQVA
481 SLRVTPNMST WRPCDQVESA VAWKYGVERQ DGPTALILSQ QNLAQQERTE EQLANIARGG
541 YVLKDCAGQP ELIFIATGSE VELAVAAYEK LTAEGVKARV VSMPSTDAFD KQDAAYRESV
601 LPKAVTARVA VEAGIADYWY KYVGLNGAIV GMTTFGESAP AELLFEEFGF TVDNVVAKAK
661 ELL
Nucleotide sequences of E. coli transketolase (i.e., E.coli transketolase mutant TK/D469T/R520Q/S385Y) (SEQ ID NO: 17) 
atgtcctcacgtaaagagcttgccaatgctattcgtgcgctgagcatggacgcagtacagaaagccaaatccggtcacccgggtg
cccctatgggtatggctgacattgccgaagtcctgtggcgtgatttcctgaaacacaacccgcagaatccgtcctgggctgaccgt
gaccgcttcgtgctgtccaacggccacggctccatgctgatctacagcctgctgcacctcaccggttacgatctgccgatggaag
aactgaaaaacttccgtcagctgcactctaaaactccgggtcacccggaagtgggttacaccgctggtgtggaaaccaccaccg
gtccgctgggtcagggtattgccaacgcagtcggtatggcgattgcagaaaaaacgctggcggcgcagtttaaccgtccgggcc
acgacattgtcgaccactacacctacgccttcatgggcgacggctgcatgatggaaggcatctcccacgaagtttgctctctggcg
ggtacgctgaagctgggtaaactgattgcattctacgatgacaacggtatttctatcgatggtcacgttgaaggctggttcaccgac
gacaccgcaatgcgtttcgaagcttacggctggcacgttattcgcgacatcgacggtcatgacgcggcatctatcaaacgcgcag
tagaagaagcgcgcgcagtgactgacaaaccttccctgctgatgtgcaaaaccatcatcggtttcggttccccgaacaaagccgg
tacccacgactcccacggtgcgccgctgggcgacgctgaaattgccctgacccgcgaacaactgggctggaaatatgcgccgtt
cgaaatcccgtctgaaatctatgctcagtgggatgcgaaagaagcaggccaggcgaaagaatccgcatggaacgagaaattcg
ctgcttacgcgaaagcttatccgcaggaagccgctgaatttacccgccgtatgaaaggcgaaatgccgtctgacttcgacgctaa
agcgaaagagttcatcgctaaactgcaggctaatccggcgaaaatcgccagccgtaaagcgtctcagaatgctatcgaagcgttc
ggtccgctgttgccggaattcctcggcggttctgctgacctggcgccgtataacctgaccctgtggtctggttctaaagcaatcaac
gaagatgctgcgggtaactacatccactacggtgttcgcgagttcggtatgaccgcgattgctaacggtatctccctgcacggtgg
cttcctgccgtacacctccaccttcctgatgttcgtggaatacgcacgtaacgccgtacgtatggctgcgctgatgaaacagcgtca
ggtgatggtttacacccacgactccatcggtctgggcgaaaccggcccgactcaccagccggttgagcaggtcgcttctctgcgc
gtaaccccgaacatgtctacatggcgtccgtgtgaccaggttgaatccgcggtcgcgtggaaatacggtgttgagcgtcaggacg
gcccgaccgcactgatcctctcccaacagaacctggcgcagcaggaacgaactgaagagcaactggcaaacatcgcgcgcgg
tggttatgtgctgaaagactgcgccggtcagccggaactgattttcatcgctaccggttcagaagttgaactggctgttgctgccta
cgaaaaactgactgccgaaggcgtgaaagcgcgcgtggtgtccatgccgtctaccgacgcatttgacaagcaggatgctgctta
ccgtgaatccgtactgccgaaagcggttactgcacgcgttgctgtagaagcgggtattgctgactactggtacaagtatgttggcct
gaacggtgctatcgtcggtatgaccaccttcggtgaatctgctccggcagagctgctgtttgaagagttcggcttcactgttgataa
cgttgttgcgaaagcaaaagaactgctgtaa
Amino acid sequences of transaminase ATA117 (SEQ ID NO: 18) 
1 MAFSADTSEI VYTHDTGLDY ITYSDYELDP ANPLAGGAAW IEGAFVPPSE ARISIFDQGY
61 LHSDVTYTVF HVWNGNAFRL DDHIERLFSN AESMRIIPPL TQDEVKEIAL ELVAKTELRE
121 AFVSVSITRG YSSTPGERDI TKHRPQVYMY AVPYQWIVPF DRIRDGVHAM VAQSVRRTPR
181 SSIDPQVKNF QWGDLIRAVQ ETHDRGFEAP LLLDGDGLLA EGSGFNVVVI KDGVVRSPGR
241 AALPGITRKT VLEIAESLGH EAILADITLA ELLDADEVLG CTTAGGVWPF VSVDGNPISD
301 GVPGPVTQSI IRRYWELNVE SSSLLTPVQY
Nucleotide sequences of transaminase ATA117 (SEQ ID NO: 19) 
atggcatttagcgcggataccagcgaaatcgtctatacccacgataccggtctggattacatcacctacagcgattacgaactggatc
cggcaaatccgttagcaggcggcgcagcttggattgaaggcgcatttgttccgccgtctgaagcgcgtattagcatcttcgatcagg
gctatctgcatagcgacgttacctataccgtcttccacgtttggaacggtaacgcatttcgtctggacgatcacatcgaacgtctgttca
gcaacgccgaaagtatgcgtattattccgccgctgacccaagacgaagtcaaagaaatcgcgctggaactggttgcaaaaaccga
actgcgcgaagcgtttgttagcgttagcattacccgcggttatagtagtaccccgggcgaacgcgatattaccaaacatcgtccgca
ggtctacatgtacgctgttccgtaccagtggatcgttccgtttgatcgtattcgcgacggcgttcacgcaatggttgcacagagcgtg
cgtcgtaccccgcgtagcagcatcgatccgcaggtcaaaaacttccagtggggcgatttaattcgcgcagttcaggaaacccacga
tcgcggttttgaagcaccgttactgttagacggcgacggtttactggcagaaggtagcggctttaacgtcgtcgtcattaaagatggg
gttgttcgttctccgggtcgtgcagcattaccgggtattacccgcaaaaccgttctggaaattgcggaatccctgggtcatgaagcga
ttctggcggatattaccttagcggaactgctggacgcagacgaagttttaggttgtaccaccgctggcggcgtttggccgtttgttagc
gttgacggcaatccgattagcgatggtgttccgggtccggttacccaaagcattattcgtcgctactgggagctgaacgttgaaagta
gcagcctgttaaccccggttcagtat
 The above provides a detailed description of preferred Embodiments of the present invention. It should be noted that numerous adjustments and alterations to common technologies in this sector can be accomplished based on the concept of the present invention without using creative work. Therefore, any technical solution that can be obtained by technical personnel in the present technical field based on the concept of the present invention and existing technologies through logical analysis, reasoning, or limited experiments should be protected within the scope determined by the claims.

## Claims

1. A high-stereoselectivity *R* transketolase mutant, which is **characterized in that** its amino acid sequence is that represented by SEQ ID NO: 1.

2. The high-stereoselectivity R transketolase mutant is **characterized in that** the nucleotide sequence of its encoding gene represented by SEQ ID NO: 2, as defined in Claim 1.

3. The recombinant expression vector containing the encoding gene of the high-stereoselectivity *R* transketolase mutant as defined in Claim 2.

4. The host cells with the recombinant expression vector containing the encoding gene of a high-stereoselectivity *R* transketolase mutant as defined in Claim 3.

5. A method for preparing the high-stereoselectivity *R* transketolase mutant, as defined in Claim 1, is **characterized in that** the said method includes the following steps:
Step 1: With the gene derived from prokaryotic E. coli transketolase as a template, obtain the high-stereoselectivity *R* transketolase mutant DNA molecules through PCR site-directed mutation and saturation mutation;
Step 2: Construct a recombinant expression vector containing the high-stereoselectivity *R* transketolase mutant DNA molecules obtained according to the Step 1;
Step 3: Through IPTG induction, produce and obtain a large amount of high-stereoselectivity *R* transketolase mutant with biological activity from the host cells containing the recombinant expression vector obtained according to the Step 2;
Step 4: Obtain high activity and stereoselectivity R ransketolase mutant protein through affinity chromatography by separating and purifying the high-stereoselectivity *R* transketolase mutant protein with biological activity obtained according to the Step 3.

6. A method for preparing a high-stereoselectivity *R* transketolase mutant, as defined in Claim 5, which is **characterized in that** the said transketolase mutant, is generated by the amino acid sequence of the said prokaryotic E. coli transketolase through the said site-directed mutation and iterative saturation mutation; The serine at Site 385 of the amino acid sequence of the said prokaryotic E. coli transketolase is mutated into tyrosine; The aspartic acid at Site 469 is mutated into threonine; The arginine at Site 520 is mutated into glutamine; The histidine at Site 26 is mutated into tyrosine; The phenylalanine at Site 434 is mutated into tyrosine; The leucine at Site 466 is mutated into histidine.

7. The application of the high-stereoselectivity transketolase mutant, as defined in Claim 1, catalyzes the formation of high-stereoselectivity *R*-aromatic dihydroxyketone from aromatic aldehyde.

8. The application of the Transketolase's encoding gene mutant, as defined in Claim 2 catalyzes the formation of high-stereoselectivity *R*-aromatic dihydroxyketone from aromatic aldehydes.

9. The application defined in Claim 7, which is **characterized in that** that with p-methyl sulfonyl benzaldehyde as the substrate and said transketolase mutant as the catalytic enzyme, the said p-methyl sulfonyl benzaldehyde could be synthesized into *R*-p-methyl sulfonyl phenyl dihydroxyketone.

10. The application defined in Claim 8 is **characterized in that** a recombinant expression vector containing the encoding gene of the high-stereoselectivity *R* transketolase mutant that can be constructed; The host cells containing the said recombinant expression vector can produce a large amount of high-stereoselectivity *R* transketolase mutant with biological activity through IPTG induction; The high-stereoselectivity R transketolase mutant protein with biological activity can be separated and purified through affinity chromatography to obtain the said high activity and stereoselectivity *R* transketolase mutant protein; With the p-methyl sulfonyl benzaldehyde as the substrate and the said high activity and stereoselectivity *R* transketolase mutant protein as the catalytic enzyme, the said p-methyl sulfonyl benzaldehyde could be synthesized into the said *R*-p-methyl sulfonyl phenyl dihydroxyketone.

11. An E. coli transketolase mutant, which is **characterized in that** the said E. coli transketolase mutant has amino acid mutation sequence as represented by SEQ ID NO: 16, and the said amino acid mutation Site is H26Y or one of the following combinatorial mutation Sites: H26Y+F434G, H26Y+F434A, H26Y+F434L, H26Y+F434I, H26Y+F434V, H26Y+F434P, H26Y+F434M, H26Y+F434W, H26Y+F434S, H26Y+F434Q, H26Y+F434T, H26Y+F434C, H26Y+F434N, H26Y+F434Y, H26Y+F434D, H26Y+F434E, H26Y+F434K, H26Y+F434R, H26Y+F434H, H26Y+F434Y+L466F, H26Y+F434Y+L466G, H26Y+F434Y+L466A, H26Y+F434Y+L466I, H26Y+F434Y+L466V, H26Y+F434Y+L466P, H26Y+F434Y+L466M, H26Y+F434Y+L466W, H26Y+F434Y+L466S, H26Y+F434Y+L466Q, H26Y+F434Y+L466T, H26Y+F434Y+L466C, H26Y+F434Y+L466N, H26Y+F434Y+L466Y, H26Y+F434Y+L466D, H26Y+F434Y+L466E, H26Y+F434Y+L466K, H26Y+F434Y+L466R, H26Y+F434Y+L466H, H26Y+F434Y+L466H+H261F, H26Y+F434Y+L466H+H261G, H26Y+F434Y+L466H+H261A, H26Y+F434Y+L466H+H261L, H26Y+F434Y+L466H+H261I, H26Y+F434Y+L466H+H261V, H26Y+F434Y+L466H+H261P, H26Y+F434Y+L466H+H261M, H26Y+F434Y+L466H+H261W, H26Y+F434Y+L466H+H261S, H26Y+F434Y+L466H+H261Q, H26Y+F434Y+L466H+H261T, H26Y+F434Y+L466H+H261C, H26Y+F434Y+L466H+H261N, H26Y+F434Y+L466H+H261Y, H26Y+F434Y+L466H+H261D, H26Y+F434Y+L466H+H261E, H26Y+F434Y+L466H+H261K, H26Y+F434Y+L466H+H261R, H26Y+F434Y+L466H+H461F, H26Y+F434Y+L466H+H461G, H26Y+F434Y+L466H+H461A, H26Y+F434Y+L466H+H461L, H26Y+F434Y+L466H+H461I, H26Y+F434Y+L466H+H461V, H26Y+F434Y+L466H+H461P, H26Y+F434Y+L466H+H461M, H26Y+F434Y+L466H+H461W, H26Y+F434Y+L466H+H461S, H26Y+F434Y+L466H+H461Q, H26Y+F434Y+L466H+H461T, H26Y+F434Y+L466H+H461C, H26Y+F434Y+L466H+H461N, H26Y+F434Y+L466H+H461Y, H26Y+F434Y+L466H+H461D, H26Y+F434Y+L466H+H461E, H26Y+F434Y+L466H+H461K, H26Y+F434Y+L466H+H461R.
